# EUROPÄISCHE PATENTSCHRIFT

(11) **EP 1 972 322 B1**
(45) Veröffentlichungstag und Bekanntmachung des Hinweises auf die Patenterteilung: **01.02.2012**
(21) Anmeldenummer: 08102740.1
(22) Anmeldetag: 19.03.2008
(51) Int. Cl.: A61K 6/04

(54) **Verfahren zum Polieren von metallischen Zahnrekonstruktionen**
Method for polishing metallic dental reconstructions
Procédé de polissage de reconstructions dentaires métalliques

(30) Priorität: 19.03.2007 DE 102007013638
(43) Veröffentlichungstag der Anmeldung: 24.09.2008
(73) Patentinhaber: DeguDent GmbH, 63457 Hanau (DE)
(72) Erfinder: Fecher, Stefan, 63867, Johannesberg (DE); Haizmann, Martin, 63695, Glauburg (DE); Völkl, Dr., Lothar, 63773, Goldbach (DE); Weisensel, Lars, 63867, Johannesberg (DE)
(74) Vertreter: Stoffregen, Hans-Herbert

(56) Entgegenhaltungen:
- WO-A1-03/093527
- DE-A1- 10 207 632
- DE-C1- 3 319 457

## Beschreibung

Die Erfindung bezieht sich auf ein Verfahren zum Herstellen einer metallischen Zahnrekonstruktion.

Aus der DE-A-101 36 997 ist eine Kobalt-Dentallegierung mit mindestens einem Edelmetall bekannt, die zur Herstellung von Zahnrekonstruktionen bestimmt ist. Dieser wird durch Gießen der Dentallegierung hergestellt. Zur Erhöhung der Feinkörnigkeit der Legierung können Begleitmetalle wie Gallium, Germanium, Indium, Zinn und/oder Aluminium beigegeben werden. Hierdurch wird die Sprödigkeit gesenkt und die Korrosionsbeständigkeit erhöht. Entsprechende Rekonstruktionen können mit Kunststoff oder Keramik verblendet werden.

Um die Oberfläche nach dem Gießen zu glätten, gelangen übliche mechanische Polierverfahren zur Anwendung.

Die DE-B-944 692 bezieht sich auf ein anodisches Bad zum Polieren von Metallen, das Alkalicitrat oder Ammoniumcitrat und Wasser enthält, wobei der pH-Wert zwischen 4 bis 7 liegt.

Aus der DE-B-225 873 ist ein Verfahren zum elektrolytischen Behandeln von Metalloberflächen bekannt, bei dem zwischen den sich in dem Elektrolyten befindlichen Elektroden eine Spannung von maximal 2 V bis 3 V anliegt.

Ein Gemisch aus Schwefelsäure, Wasser und Glycerin wird als Elektrolyt benutzt, um rostfreien Eisen- und Stahllegierungen ein glänzendes Aussehen zu verleihen (DE-B-682248).

Gegenstand der DD-A-238 074 ist ein Verfahren zum Hochglänzen stromleitender Werkstücke im anodischen Elektrolytplasma. Bei den Werkstücken handelt es sich um solche, die aus mehrphasigen Legierungen bestehen.

Eine elektrolytische Glänzbehandlung von Kobalt-Chrom- oder Titan-Legierungen ist aus der DE-C-3210 315 bekannt.

Das Plasmapolieren von Titan und Titanlegierungen wird in der DE-A-102 07 632 beschrieben. Als Elektrolytlösung wird eine solche mit einem pH-Wert von 5,0 bis 7,0 benutzt.

Aus der EP-A-1 568 472 ist ein Freiform-Sintern und/oder -Schmelzen zu entnehmen, um Dentalprodukte herzustellen. Dabei wird ein Laser- oder Elektronenstrahl derart geführt, dass Positionen der jeweils auszubildenden Werkstoffschicht mehrfach bestrahlt werden, um insgesamt Fertigungszeiten zu reduzieren.

Aus der DE-C-33 19 457 ist eine Kobalt-Chrom-Legierung bekannt, die als Aufbrenn-Guss-Legierung für einen festsitzenden oder herausnehmbaren Zahnersatz bestimmt ist.

Eine weitere Kobalt-Chrom-Legierung ist der EP-A-1 696 044 zu entnehmen. Diese enthält kein Aluminium, jedoch Gallium im Bereich zwischen 2 Gew.-% und 4 Gew.-%.

Eine Dentalguss-Legierung nach der WO-A-2004/042098 weist 25 Gew.-% - 32 Gew.-% Chrom, 8 Gew.-% - 12 Gew.-% Wolfram, 0,05 Gew.-% - 0,4 Gew.-% jeweils eines oder mehrerer der Elemente in der 4. und/oder 5. Nebengruppe des Periodensystems der Elemente, herstellungsbedingte Verunreinigungen und als Rest Kobalt auf.

Gegenstand der DE-A-198 15 091 ist eine Legierung für Dentalgussteile bestehend aus 20 Gew.-% - 35 Gew.-% Chrom, 4 Gew.-% - 8 Gew.-% Molybdän, bis zu 3 Gew.-% Silizium, 0,05 Gew.-% - 1,2 Gew.-% Tantal, Niob und/oder Wolfram, wobei der Anteil jedes einzelnen Elementes Tantal, Niob bzw. Wolfram weniger als 0,5 Gew.-% beträgt, bis zu 0,3 Gew.-% Kohlenstoff, 0,05 - 0,4 Gew.-% Stickstoff, bis zu 3 Gew.-% Eisen, bis zu 3 Gew.-% Mangan, weniger als 1 Gew.-% an eventuellen Verunreinigungen und als Rest Kobalt.

Die WO-A-03/093527 bezieht sich auf ein Verfahren zur Oberflächenmodifizierung einer Kobalt-Chrom-Legierung, die als Zahnrekonstruktion verwendet werden kann.

Der vorliegenden Erfindung liegt die Aufgabe zugrunde, ein Verfahren der eingangs genannten Art so weiterzubilden, dass reproduzierbar eine definierbare Flächenrauigkeit erzielbar ist. Dabei soll die Legierung ein gutes Verarbeitungsverhalten zeigen. Die Bruchneigung ist zu minimieren, wobei Härtewerte erzielt werden sollen, die eine problemlose Nachbearbeitung ermöglichen. Auch sollen Korrosionsraten erzielbar sein, die im Bereich von Edelmetallen liegen.

Zur Lösung der Aufgabe wird vorgeschlagen, ein Verfahren zum Herstellen einer metallischen Zahnrekonstruktion, wie Gerüst, unter Verwendung einer Kobalt-Chrom-Legierung mit einer Zusammensetzung von
43 - 68 Gew.-% Kobalt,
12 - 30 Gew.-% Chrom,
8 - 25 Gew.-% Wolfram,
0 - 13 Gew.-% Eisen,
0 - 30 Gew.-% Mangan,
0 - 10 Gew.-% Molybdän,

0-5 Gew.-% zumindest eines der Elemente Aluminium, Tantal, Rhenium, Titan und weniger als 0,1 Gew.-% Kohlenstoff, wobei die Kobalt-Chrom-Legierung in Pulverform verdüst und der Zahnersatz im Rapid Prototyping-Verfahren hergestellt und anschließend durch Plasmapolieren poliert wird

Überraschenderweise hat die Kobalt-Chrom-Legierung zuvor genannter Zusammensetzung zu einem durch Laser-Schmelz- und/oder -Sinter-Verfahren, also durch FreiformSchmelzen bzw. Sintern hergestellten Zahnersatz geführt, der Bruchdehnungseigenschaften aufweist, wie diese von Gusslegierungen bekannt sind. Gleichzeitig ergibt sich jedoch überraschenderweise eine Korrosionsrate von weniger als 10 µg/cm² in 7 Tagen gemessen nach der DIN 22674, die um mehr als einen Faktor 6 unter dem Korrosionswert einer Dentalguss-Legierung gleicher Zusammensetzung liegt.

Die Korrosionsrate nach DIN 22674 wird dadurch bestimmt, dass ein Plättchen der Legierung - hergestellt durch Gießen bzw. durch Laser-Schmelz- und/oder Sinter-Verfahren - einer Prüflösung ausgesetzt wird, die der ISO 10271 entspricht, um sodann die Menge der aus dem Plättchen herausgelösten Materialien nach 7 Tagen zu bestimmen, wobei die Fläche des Plättchens bekannt ist.

Neben der hervorragenden Korrosionsbeständigkeit sind gleichfalls die guten mechanischen Eigenschaften überraschend. Beides kann möglicherweise dadurch erklärt werden, dass die Kinetik der intermetallischen Phasen durch das Laser-Schmelzen bzw. -Sintern dahingehend beeinflusst wird, dass sich die Phasen im Vergleich zum Gießen nicht derart ausbilden können, dass sich die hierdurch bedingten negativen Eigenschaften bemerkbar machen.

Des Weiteren ergibt sich ein Zahnersatz, der vor einer weiteren Wärmebehandlung eine Härte von weniger als 350 (HV 10) aufweist, so dass eine gute Nachbearbeitung möglich ist. Sofern jedoch eine Wärmebehandlung erforderlich ist, um Verzüge auszugleichen, haben Messungen ergeben, dass die Härte zwar auf über 400 (HV 10) ansteigt, ungeachtet dessen weiterhin eine gute Nachbearbeitung möglich ist, eine Eigenschaft, die nicht vorhersehbar war.

Insbesondere ergeben sich die positiven Eigenschaften des Zahnersatzes bzw. Gerüstes dann, wenn eine Herstellung durch Laser-Schmelzen erfolgt, also die einzelnen Pulverschichten nacheinander vollständig aufgeschmolzen werden. Auf die jeweils aufgeschmolzene und sodann erstarrte Schicht wird eine weitere Pulverschicht aufgetragen, die folglich gleichfalls vollständig aufgeschmolzen und dabei an die darunter liegende Schicht angeschmolzen wird. Diese Verfahrensschritte werden sukzessiv durchgeführt, um so durch Freiformschmelzen die gewünschte Geometrie des herzustellenden Zahnersatzes bzw. Gerüstes zu erzielen.

Insbesondere besteht die Möglichkeit, dass die Legierung 50 Gew.-% - 65 Gew.-% Kobalt, 15 Gew.-% - 22 Gew.-% Chrom, 15 Gew.-% - 22 Gew.-% Wolfram, 4 Gew.-% - 11 Gew.-% Eisen und 1 Gew.-% - 3 Gew.-% Aluminium enthält.

Die Kobalt-Chrom Legierung kann Wolfram und/oder Molybdän mit einem Gehalt zwischen 8 Gew.-% und 35 Gew.-% enthalten.

Zusätzlich können in der Kobalt Chrom-Legierung 0 Gew.-% - 0,2 Gew.-% zumindest eines der Elemente Bor, Yttrium, 0 Gew.-% - 2 Gew.-% zumindest eines der Elemente Vanadium, Silizium, Kupfer, Zink, Niob, 0 Gew.-% - 10 Gew.-% Gallium, 0 Gew.-% - 5 Gew.-% Germanium und 0 Gew.-% - 1 Gew.-% zumindest eines der Elemente Cer, Lanthan enthalten sein.

Allerdings sollte die Legierung nicht mehr als 50 Gew.-% Chrom, Wolfram und Rhenium enthalten.

Bevorzugterweise liegt das Verhältnis Chrom : Wolfram + Rhenium zwischen ca. 2:3 und ca. 2:1 bzw. zwischen ca. 2:3 und ca. 3:2.

Der Gehalt von Chrom + Wolfram ist mindestens 30 Gew.-% und höchstens 50 Gew.-% und das Verhältnis von Chrom : Wolfram liegt zwischen ca. 3:4 und ca. 4:3.

Bezüglich der Zusammensetzung des Elektrolyten und dessen pH-Wert-Einstellung oder die Parameter, die beim Plasmapolieren bevorzugterweise eingehalten werden sollten, wird auf die zuvor erfolgten Ausführungen verwiesen.

Erfindungsgemäß wird eine Zahnrekonstruktion mittels Plasmapolierens geglättet, um eine definierte Oberfläche zu erhalten, die sodann für den Fall, dass eine Verblendung erfolgen soll, gezielt z. B. durch Sandstrahlen aufgeraut wird. Soll eine Verblendung nicht erfolgen, so bietet sich eine überaus glatte Fläche, die zur Erhöhung der Korrosionsbeständigkeit fuhrt.

Optimale Ergebnisse ergeben sich dann, wenn das Plasmapolieren bei einer Stromdichte von 0,25 A/cm² bis 0,5 A/cm² durchgeführt wird. Die Spannung sollte zwischen 300 V und 380 V liegen, wobei die Zahnrekonstruktion beim Plasmapolieren Anode ist.

Zur Optimierung des Verfahrens sieht die Erfindung vor, dass der Elektrolyt zu Beginn des Plasmapolierens auf eine Temperatur T mit T_{S} - 2°C ≥ T eingestellt wird, wobei T_{S} = Siedetemperatur des Elektrolyten ist. Insbesondere wird der Elektrolyt auf eine Temperatur 80 °C ≤ T ≤ T_{S} - 2°C eingestellt.

Insbesondere sollte das Plasmapolieren über einen Zeitraum bis maximal 15 Minuten, bevorzugterweise weniger als 10 Minuten durchgeführt werden.

Das erfindungsgemäße Polieren stellt insbesondere bei Zahnrekonstruktionen, die durch das Lasersintem- und/oder -schmelzverfahren hergestellt werden, sicher, dass die herstellungsbedingten Oberflächenunebenheiten abgetragen werden derart, dass sich eine amorphe, glasartige Oberflächenschicht mit geringer spezifischer Oberfläche ergibt. Dies erhöht die Korrosionsbeständigkeit wegen der geringeren Ionenabgabe an die Umgebung im Vergleich zu Oberflächen, die mechanisch poliert werden.

Besonders bevorzugt wird das erfindungsgemäße Verfahren für metallische Zahnrekonstruktionen eingesetzt, die nicht durch Gießen, sondern durch Lasersinter- bzw. -schmelz-Verfahren hergestellt werden, also nach einem solchen, wie dieses prinzipiell in der DE-C-196 49 865 beschrieben wird, auf deren Offenbarung verwiesen wird.

Mit anderen Worten gelangt ein Rapid Prototyping-Verfahren zur Anwendung, bei dem das Legierungspulver nacheinander schichtweise übereinander aufgebracht und jede Pulverschicht vor dem Aufbringen der nächstliegenden Pulverschicht mit einem fokussierten Laserstrahl in einem vorgegebenen Bereich, der einem ausgewählten Querschnittsbereich der Zahnkonstruktion entspricht, auf eine vorgegebene Temperatur derart erhitzt wird, dass die Pulverschicht an der darunter liegenden Pulverschicht durch Aufschmelzen der Pulverschicht und/oder Sintern fixiert wird.

## Patentansprüche

1. Verfahren zum Herstellen einer metallischen Zahnrekonstruktion, wie Gerüst, unter Verwendung einer Kobalt-Chrom-Legierung mit einer Zusammensetzung von
43 - 68 Gew.-% Kobalt,
12 - 30 Gew.-% Chrom,
8 - 25 Gew.-% Wolfram,
0 -13 Gew.-% Eisen,
0 - 30 Gew.-% Mangan,
0 -10 Gew.-% Molybdän,
0 - 5 Gew.-% zumindest eines der Elemente Aluminium, Tantal, Rhenium, Titan und weniger als 0,1 Gew.-% Kohlenstoff, wobei die Kobalt-Chrom-Legierung in Pulverform verdüst und der Zahnersatz im Rapid Prototyping-Verfahren hergestellt und anschließend durch Plasmapolieren poliert wird.

2. Verfahren nach Anspruch 1,
**dadurch gekennzeichnet,**
**dass** die Legierung 50 Gew.-% - 65 Gew.-% Kobalt, 15 Gew.-% - 22 Gew.-% Chrom, 15 Gew.-% - 22 Gew.-% Wolfram, 4 Gew.-% - 11 Gew.-% Eisen und 1 Gew.-% - 3 Gew.-% Aluminium enthält.

3. Verfahren nach Anspruch 1,
**dadurch gekennzeichnet,**
**dass** die Zahnkonstruktion während des Plasmapolierens in einen Elektrolyten in Form eines Gemisches aus Wasser und zumindest einem Ammoniumsalz wie Ammoniumsulfat oder Ammoniumhydrogensulfat eingetaucht wird und der Elektrolyt auf einen pH-Wert mit 1 ≤ pH ≤ 3 insbesondere durch Beigabe von Schwefelsäure eingestellt wird.

4. Verfahren nach Anspruch 1,
**dadurch gekennzeichnet,**
**dass** das Plasmapolieren bei einer Stromdichte von 0,25 A/cm² bis 0,5 A/cm² und/oder bei einer zwischen der Zahnrekonstruktion als Anode und einer Kathode angelegten Spannung U mit 300V ≤ U ≤ 380V durchgeführt wird.

5. Verfahren nach Anspruch 1,
**dadurch gekennzeichnet,**
**dass** der Elektrolyt zu Beginn des Plasmapolierens auf eine Temperatur T mit T_{S} -2 °C ≥ T, insbesondere mit 80 °C ≤ T ≤ T_{S} -2 °C eingestellt wird, wobei T_{S} = Siedetemperatur des Elektrolyten ist.

6. Verfahren nach Anspruch 1,
**dadurch gekennzeichnet,**
**dass** die Zahnrekonstruktion über eine Zeit t mit t ≤ 15 min., vorzugsweise t ≤ 10 min. plasmapoliert wird.

7. Verfahren nach Anspruch 1,
**dadurch gekennzeichnet,**
**dass** die metallische Zahnrekonstruktion durch Lasersinter- und/oder -schmelz-Verfahren hergestellt wird.

8. Verfahren nach Anspruch 1,
**dadurch gekennzeichnet,**
**dass** die Cobalt-Chrom-Legierung Wolfram und Molybdän mit einem Gehalt zwischen 8 Gew.-% und 35 Gew.-% enthält.

9. Verfahren nach Anspruch 1,
**dadurch gekennzeichnet,**
**dass** die Kobalt-Chrom-Legierung zusätzlich 0 Gew.-% - 0,2 Gew.-% zumindest eines der Elemente Bor, Yttrium, 0 Gew.-% - 2 Gew.-% zumindest eines der Elemente Vanadium, Silizium, Kupfer, Zink, Niob, 0 Gew.-% - 10 Gew.-% Gallium, 0 Gew.-% - 5 Gew.-% Germanium und 0 Gew.-% - 1 Gew.-% zumindest eines der Elemente Cer, Lanthan enthält.

10. Verfahren nach Anspruch 1,
**dadurch gekennzeichnet,**
**dass** die Legierung nicht mehr als 50 Gew.-% Chrom, Wolfram und Rhenium enthält, dass vorzugsweise das Verhältnis Chrom : Wolfram + Rhenium zwischen ca. 2:3 und ca. 2:1 bzw. zwischen ca. 2:3 und ca. 3:2 liegt, insbesondere dass der Gehalt von Chrom + Wolfram mindestens 30 Gew.-% und höchstens 50 Gew.-% ist und das Verhältnis von Chrom : Wolfram zwischen ca. 3:4 und ca. 4:3 liegt.

## Claims

1. Method for the manufacture of a metallic dental prosthesis, such as a frame, through the use of a cobalt-chromium alloy with a composition of 43% to 68% by weight of cobalt,
12% to 30% by weight of chromium,
8% to 25% by weight of tungsten,
0% to 13% by weight of iron,
0% to 30% by weight of manganese,
0% to 10% by weight of molybdenum,
0% to 5% by weight of at least one of the elements aluminum, tantalum, rhenium, titanium, and less than 0.1% by weight of carbon, whereby the cobalt-chromium alloy is sprayed in powder form and the dental prosthesis is manufactured by means of a rapid prototyping process and subsequently polished by plasma polishing.

2. Method according to claim 1,
**characterized in**
**that** the alloy contains 50% to 65% by weight of cobalt, 15% to 22% by weight of chromium, 15% to 22% by weight of tungsten, 4% to 11 % by weight of iron and 1% to 3% by weight of aluminum.

3. Method according to claim 1,
**characterized in**
**that** the dental prosthesis, during the plasma polishing process, is immersed in an electrolyte in the form of a mixture of water and at least one ammonium salt such as ammonium sulfate or ammonium hydrogen sulfate and the electrolyte is set to a pH value of 1 ≤ pH ≤ 3 especially by adding sulfuric acid.

4. Method according to claim 1,
**characterized in**
**that** the plasma polishing is performed at a current density of 0.25 A/cm² to 0.5 A/cm² and/or at a voltage U of 300V ≤ U≤ 380V applied between the dental prosthesis as anode and a cathode.

5. Method according to claim 1,
**characterized in**
**that** the electrolyte, at the beginning of the plasma polishing, is set to a temperature T, where T_{S} -2°C ≥ T, especially with 80°C ≤ T ≤ T_{S} -2°C, whereby T_{S} = boiling temperature of the electrolyte.

6. Method according to claim 1,
**characterized in**
**that** the dental prosthesis is plasma-polished over a time t where t ≤ 15 min, preferably t ≤ 10 min.

7. Method according to claim 1,
**characterized in**
**that** the metallic dental prosthesis is manufactured by means of a laser sintering and/or laser melting process.

8. Method according to claim 1,
**characterized in**
**that** the cobalt-chrome alloy contains tungsten and molybdenum, with a content between 8% and 35% by weight.

9. Method according to claim 1,
**characterized in**
**that** the cobalt-chrome alloy additionally contains 0% to 0.2% by weight of at least one of the elements boron and yttrium, 0% to 2% by weight of at least one of the elements vanadium, silicon, copper, zinc, niobium, 0% to 10% by weight of gallium, 0% to 5% by weight of germanium and 0% to 1% by weight of at least one of the elements cerium, lanthanum.

10. Method according to claim 1,
**characterized in**
**that** the alloy does not contain more than 50% by weight of chromium, tungsten and rhenium, that preferably the ratio of chromium : tungsten + rhenium is between approximately 2:3 and approximately 2:1, respectively between approximately 2:3 and approximately 3:2, especially that the content of chromium + tungsten is at least 30% by weight and at most 50% by weight, and the ratio of chromium : tungsten is between approximately 3:4 and approximately 4:3.

## Revendications

1. Procédé de fabrication d'une reconstruction dentaire métallique, telle qu'une armature, utilisant un alliage chrome-cobalt composé de 43 à 68 % en poids de cobalt,
12 à 30 % en poids de chrome,
8 à 25 % en poids de tungstène,
0 à 13 % en poids de fer,
0 à 30 % en poids de manganèse,
0 à 10 % en poids de molybdène,
0 à 5 % en poids d'au moins un des éléments aluminium, tantale, rhénium, titane et moins de 0,1 % en poids de carbone, sachant que l'alliage cobalt-chrome est atomisé en poudre et que la prothèse dentaire est fabriquée par procédé de prototypage rapide puis polie au plasma.

2. Procédé selon la revendication 1,
**caractérisé en ce**
**que** l'alliage contient 50 à 65 % en poids de cobalt, 15 à 22 % en poids de chrome, 15 à 22 % en poids de tungstène, 4 à 11 % en poids de fer et 1 à 3 % en poids d'aluminium.

3. Procédé selon la revendication 1,
**caractérisé en ce**
**que**, pendant le polissage au plasma, la construction dentaire est plongée dans un électrolyte formé d'un mélange d'eau et d'au moins un sel d'ammonium tel que le sulfate d'ammonium ou l'hydrogénosulfate d'ammonium, et que l'électrolyte est réglé sur une valeur pH de 1 ≤ pH ≤ 3, en particulier par adjonction d'acide sulfurique.

4. Procédé selon la revendication 1,
**caractérisé en ce**
**que** le polissage au plasma est réalisé à une densité de courant de 0,25 A/cm² à 0,5 A/cm² et/ou à une tension U de 300 V ≤ U ≤ 380 V appliquée entre la construction dentaire en tant qu'anode et une cathode.

5. Procédé selon la revendication 1,
**caractérisé en ce**
**que** l'électrolyte est réglé à une température T de Ts -2 °C ≥ T, en particulier de 80 °C ≤ T ≤ Ts -2 °C au début du polissage au plasma, sachant que Ts = température d'ébullition de l'électrolyte.

6. Procédé selon la revendication 1,
**caractérisé en ce**
**que** la reconstruction dentaire est polie au plasma pendant une durée t de t ≤ 15 min., de préférence de t ≤ 10 min.

7. Procédé selon la revendication 1,
**caractérisé en ce**
**que** la reconstruction dentaire métallique est fabriquée par procédé de frittage au laser et/ou de fusion au laser.

8. Procédé selon la revendication 1,
**caractérisé en ce**
**que** l'alliage cobalt-chrome contient du tungstène et du molybdène à un taux compris entre 8 et 35 % en poids.

9. Procédé selon la revendication 1,
**caractérisé en ce**
**que** l'alliage cobalt-chrome contient en outre 0 à 0,2 % en poids d'au moins un des éléments bore, yttrium, 0 à 2 % en poids d'au moins un des éléments vanadium, silicium, cuivre, zinc, niobium, 0 à 10 % en poids de gallium, 0 à 5 % en poids de germanium et 0 à 1 % en poids d'au moins un des éléments cérium, lanthane.

10. Procédé selon la revendication 1,
**caractérisé en ce**
**que** l'alliage ne contient pas plus de 50 % en poids de chrome, tungstène et rhénium, que de préférence le rapport chrome : tungstène + rhénium se situe entre env. 2:3 et env. 2:1 ou entre env. 2:3 et env. 3:2, en particulier que la part de chrome + tungstène s'élève à au moins 30 % en poids et au maximum à 50 % en poids, et que le rapport chrome : tungstène se situe entre env. 3:4 et env. 4:3.
